# FASCICULE DE BREVET EUROPEEN

(11) **EP 2 882 399 B1**
(45) Date de publication et mention de la délivrance du brevet: **12.10.2016**
(21) Numéro de dépôt: 13745667.9
(22) Date de dépôt: 06.08.2013
(51) Int. Cl.: A61H 35/04, A61M 3/02

(54) **DISPOSITIF D'IRRIGATION DES CAVITÉS NASO-SINUSIENNES**
VORRICHTUNG ZUR SPÜLUNG DER NASENNEBENHÖHLEN
DEVICE FOR IRRIGATING THE NASO-SINAL CAVITIES

(30) Priorité: 09.08.2012 FR 1257725
(43) Date de publication de la demande: 17.06.2015
(73) Titulaire: Laboratoire de la Mer, 35400 Saint-Malo (FR)
(72) Inventeur: BERTAUD, Olivier, 35400 Saint-Malo (FR); BEAULIEU, Anne, 35400 Saint-Malo (FR)
(74) Mandataire: Le Guen-Maillet
(86) Numéro de dépôt international: PCT/EP2013/066439
(87) Numéro de publication internationale: WO 2014/023713

(56) Documents cités:
- CA-A1- 2 486 719
- DE-U1-202011 050 071
- US-A1- 2010 152 703
- US-A1- 2011 166 523
- US-B1- 6 241 705

## Description

La présente invention concerne un dispositif d'irrigation des cavités naso-sinusiennes.

Les cavités naso-sinusiennes constituent une porte d'entrée pour divers microorganismes tels que des bactéries ou des virus qui peuvent alors coloniser la sphère ORL et même étendre leur progression jusque dans les bronches avec pour conséquences le développement de diverses pathologies. Les muqueuses des fosses nasales agissent comme des filtres pour faire barrière à ces agressions mais peuvent être fragilisées par exemple par le froid ou la pollution. Une solution reconnue comme efficace consiste à nettoyer par irrigation les cavités naso-sinusiennes, en particulier les fosses nasales, afin de les débarrasser des impuretés et de soutenir l'activité de nettoyage des muqueuses. L'irrigation peut être effectuée à l'aide d'un liquide prévu à cet effet tel que des solutions salines, par exemple des solutions d'eau de mer isoosmotiques ou hyperosmotiques ou bien du sérum physiologique ou bien de l'eau rendue isotonique ou hypertonique.

Un dispositif bien connu d'irrigation des cavités naso-sinusiennes est connu sous le nom de pot de Neti. Il consiste en un récipient comportant une ouverture pour son remplissage et destiné à être rempli d'un liquide prévu à cet effet. Il est pourvu d'un conduit faisant saillie du récipient et terminé par un embout destiné à être engagé dans une narine. La forme globale d'un tel dispositif rappelle une lampe d'Aladin. Le passage du liquide jusque dans la narine se fait par inclinaison du récipient de bas en haut et donc par gravité. Ce dispositif nécessite de faire pénétrer l'embout de manière perçue comme trop invasive et inconfortable. De plus, il ne permet pas de contrôler le débit du flux de liquide, ce qui peut être intéressant en fonction de la sévérité des symptômes naso-sinusiens, de la préférence ou de l'action recherchée par les utilisateurs.

D'autres dispositifs ont été proposés depuis. Par exemple, on connaît un dispositif qui consiste en un récipient comprenant une ouverture sur laquelle vient se visser un embout pourvu lui-même d'une ouverture à son sommet. Un conduit tubulaire plongeant à l'intérieur du récipient est prévu pour assurer, par ascension, le passage du liquide prévu pour l'irrigation depuis l'intérieur du récipient jusqu'à l'ouverture pratiquée au sommet de l'embout. Les parois du récipients sont souples de manière à ce que la compression des parois par une main force le liquide à l'intérieur du conduit tubulaire de manière à créer un flux de liquide depuis le récipient jusque dans les fosses nasales.

Bien qu'efficace, un tel dispositif ne permet pas l'ajustement et le contrôle du débit du flux de liquide s'écoulant jusqu'à la narine. En effet, le débit du flux ne varie qu'en fonction de la pression exercée par la main lors de la compression des parois. Un autre inconvénient de ce type de dispositif est qu'il ne permet le lavage ou le traitement des cavités naso-sinusiennes que par ascension du liquide dans un tube plongeur, dans le sens contraire à la gravité, et donc par pression. Un tel procédé d'irrigation est connu sous le nom anglais de « squeeze ». Il s'agit d'une irrigation qui se révèle intense et préférentiellement adaptée au cas de congestions fortes. Or il peut être préférable, dans certaines situations, telles qu'en cas de symptômes modérés ou de situation de prévention des pathologies des sinus ou de la sphère ORL, de procéder à des lavages moins intenses comme réalisés à l'aide d'un pot de Neti classique, c'est-à-dire écoulement du liquide par gravité.

Le document CA-A1-2486719 divulgue un dispositif d'irrigation des cavités naso-sinusiennes avec toutes les caractéristiques techniques du préambule de la revendication 1.

Il est, de plus, régulièrement observé un abandon des soins de lavage et de traitement des cavités naso-sinusiennes par les utilisateurs lorsque les dispositifs qu'ils utilisent ne permettent pas un mode d'irrigation adapté à leurs besoins ou pathologies ou lorsque ceux-ci sont peu pratiques.

En outre, il est essentiel que de tels dispositifs puissent être démontés et nettoyés aisément et efficacement afin d'éviter toute contamination bactérienne.

En ce qui concerne les dispositifs de soin et lavage par sprays, ceux-ci ne sont pas adaptés aux cas de fortes congestions ou de symptômes chroniques d'après les observations des spécialistes en oto-rhino-laryngologie lesquels privilégient les dispositifs d'irrigation des cavités naso-sinusiennes.

Un des buts de la présente invention est de proposer un dispositif d'irrigation, c'est-à-dire pour le lavage ou le traitement des cavités naso-sinusiennes, qui ne présente pas les inconvénients de l'état de la technique précités et, en particulier, qui permet de procéder, en fonction des circonstances, soit à un lavage ou un traitement intense, soit à un lavage ou un traitement modéré ou doux.

Un autre but de la présente invention est de proposer un tel dispositif d'irrigation des cavités naso-sinusiennes qui autorise une variation du débit du flux, de manière contrôlée, de liquide utilisé pour le lavage ou le traitement.

Encore un autre but de l'invention est de proposer un tel dispositif qui soit facilement et entièrement lavable, facile à démonter et à remonter, et comprenant peu de pièces à assembler.

A cet effet, l'invention concerne un dispositif d'irrigation des cavités naso-sinusiennes selon la revendication 1.

Le moyen de réglage du débit du flux de liquide permet ainsi de choisir entre une irrigation intense ou au contraire douce sans devoir utiliser différents dispositifs. Ce moyen de réglage permet de faire varier le débit et ceci de manière contrôlée.

Le moyen de réglage du débit du flux de liquide comprend un second orifice raccordé à l'orifice d'écoulement et un troisième orifice raccordé au récipient, et des moyens prévus pour faire varier les positions relatives desdits second et troisième orifices de sorte que la section de passage de liquide traversant les second et troisième orifices puisse varier.

Le troisième orifice traverse une pièce intermédiaire apte à être solidarisée avec ledit récipient.

Ladite pièce intermédiaire comporte des moyens pour réceptionner l'embout et pour permettre la rotation dudit embout relativement à ladite pièce intermédiaire.

Le second orifice et le troisième orifice sont alignés dans un même axe lorsque l'embout est monté sur ladite pièce intermédiaire, lesdits orifices étant de section allongée de manière à ce que, lorsque l'embout est entraîné en rotation, la section de passage du liquide varie, ladite section traversant les second et troisième orifices.

Du fait de la section allongée des orifices, la variation des positions des deux orifices l'un par rapport à l'autre a pour conséquence de faire varier la section de passage prévue pour un liquide au travers des orifices. Il est donc possible de réduire cette section de passage, par rapport à la section maximale offerte, de manière à diminuer le débit de liquide qui la traverse.

Selon un mode de réalisation de l'invention, les moyens prévus pour réceptionner l'embout se composent d'un logement cylindrique dans lequel peut être logée une partie cylindrique constitutive de l'embase de l'embout.

Avantageusement, des moyens d'étanchéité sont prévus entre une paroi cylindrique du logement et une paroi cylindrique de l'embase.

Selon un mode de réalisation, les moyens d'étanchéité comprennent au moins un bourrelet formé autour de la paroi cylindrique de l'embase apte à coopérer avec la paroi cylindrique constitutive dudit logement.

Préférentiellement, au moins un moyen de retenue est prévu entre l'embout et la pièce intermédiaire. Il peut s'agir d'un bourrelet formé sur la paroi du logement et contre lequel un bourrelet de la paroi de l'embase vient buter lorsque l'embout est connecté sur la pièce intermédiaire.

Selon un mode de réalisation de l'invention, le conduit qui réunit l'orifice d'écoulement et le second orifice de l'embout est droit.

Selon un autre mode de réalisation de l'invention, le conduit qui réunit l'orifice d'écoulement et le second orifice de l'embout est coudé.

Comme décrit dans ce qui suit, deux embouts permettant des procédés d'irrigation différents peuvent être utilisés sur un même dispositif conforme à l'invention. Le choix de l'embout se fera en fonction du type d'irrigation souhaité.

Selon un mode de réalisation de l'invention, ladite pièce intermédiaire comporte des moyens pour réceptionner et pour solidariser le récipient de manière étanche.

Préférentiellement, le récipient comprend un goulot pourvu d'un filetage complémentaire d'un taraudage réalisé dans la pièce intermédiaire.

Selon un mode de réalisation, le dispositif comprend un tube plongeur prévu pour être raccordé au troisième orifice et pour plonger dans ledit récipient.

Selon encore un mode de réalisation, la troisième ouverture est pratiquée dans la paroi de fond de ladite pièce intermédiaire.

Préférentiellement, ladite pièce intermédiaire est tronconique.

De manière avantageuse, le sommet de l'embout présente une surface convexe. La surface convexe est prévue pour être apposée contre la narine de l'utilisateur. Elle présente ainsi des dimensions qui l'autorisent à être apposée contre la narine d'un adulte ou d'un enfant sans être invasive.

Selon un mode de réalisation, l'embout comporte au moins un méplat entre l'embase et le sommet.

Selon un mode de réalisation, la pièce intermédiaire comporte des indications visuelles relatives au réglage du débit du flux de liquide.

Selon encore un mode de réalisation, l'embout comporte une ou plusieurs indications visuelles relatives au réglage du débit du flux de liquide.

Avantageusement, le sommet dudit embout ou au moins la partie convexe présente une surface adhérente et non collante.

Avantageusement encore, les parois dudit récipient sont souples.

L'invention concerne encore un kit comportant un dispositif conforme à l'invention et comportant un embout dont le conduit qui réunit l'orifice d'écoulement et le second orifice est droit ainsi qu'un embout dont le conduit qui réunit l'orifice d'écoulement et le second orifice est coudé.

Les caractéristiques de l'invention mentionnées ci-dessus, ainsi que d'autres, apparaîtront plus clairement à la lecture de la description suivante d'exemples de réalisation, ladite description étant faite en relation avec les dessins joints, parmi lesquels :
La Fig. 1 représente une vue éclatée d'un dispositif d'irrigation des cavités naso-sinusiennes selon un mode de réalisation de l'invention,
La Fig. 2 représente une vue de dessous d'un embout d'un dispositif de lavage ou de traitement des cavités naso-sinusiennes selon un mode de réalisation de l'invention,
La Fig. 3 représente une vue de dessus d'une pièce intermédiaire d'un dispositif de lavage ou de traitement des cavités naso-sinusiennes selon un mode de réalisation de l'invention,
La Fig. 4 représente une vue schématique d'une section de passage traversant un orifice d'un embout et d'une pièce intermédiaire d'un dispositif selon un mode de réalisation de l'invention,
La Fig. 5 représente une vue éclatée d'un dispositif d'irrigation des cavités naso-sinusiennes selon un autre mode de réalisation de l'invention.

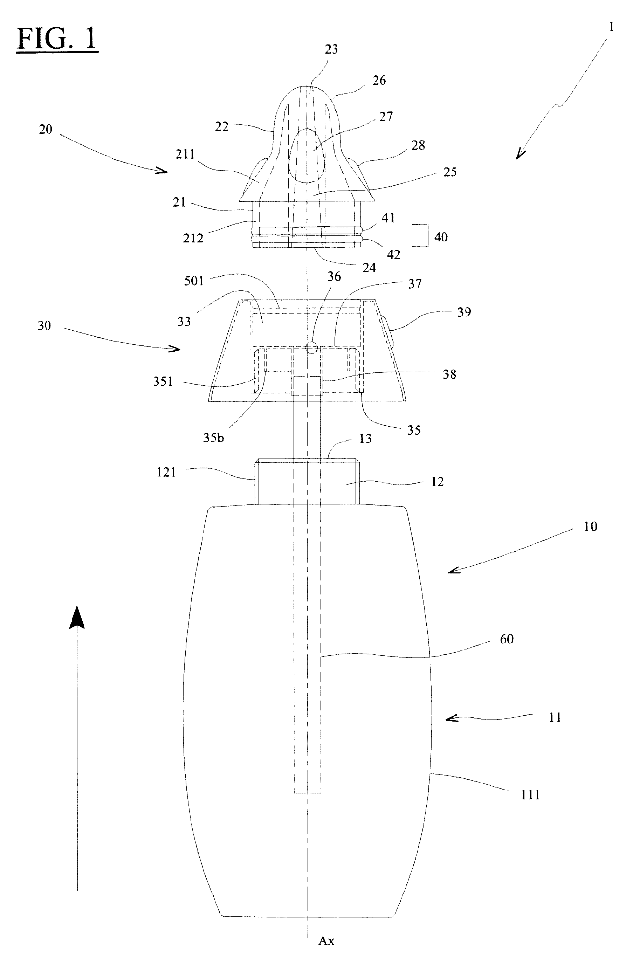

En lien avec la Fig. 1 est illustré un dispositif d'irrigation 1 des cavités naso-sinusiennes à l'aide d'un liquide prévu à cet effet, tel que de l'eau rendue isotonique ou hypertonique ou bien d'une solution saline.

Le dispositif d'irrigation 1 comprend un récipient 10 destiné à contenir le liquide prévu pour l'irrigation, et un embout 20 qui est prévu pour être fixé de manière provisoire sur le récipient 10 et qui présente un orifice 23 destiné à permettre l'écoulement du liquide hors du dispositif lors de l'irrigation.

Le dispositif d'irrigation 1 comprend, de plus, un moyen de réglage du débit du flux de liquide susceptible de s'écouler au travers de l'orifice 23.

Le moyen de réglage du débit du flux de liquide comprend un second orifice 24 qui est raccordé à l'orifice d'écoulement 23 et un troisième orifice 36 qui est raccordé au récipient 10, ainsi que des moyens prévus pour faire varier les positions relatives des second et troisième orifices 24 et 36 de sorte que la section de passage pour le liquide, au travers des orifices 24 et 36, puisse varier.

Le dispositif 1 est présenté de manière plus détaillée dans ce qui suit.

Le récipient 10 comprend un corps 11 qui est, dans ce mode de réalisation, légèrement ovoïde, comprenant un fond 14, et une ouverture 13 aménagée dans un goulot 12. Le fond 14 est préférentiellement plat de manière à permettre au récipient 10 de reposer sur son fond à la verticale.

Les parois 111 du corps 11 sont flexibles et peuvent être facilement compressées dans une main. Elles sont également transparentes ou semi opaques. Elles sont par exemples réalisées à l'aide d'un matériau plastique, préférentiellement résistant à la chaleur. Sur les parois 111 peuvent figurer, de manière connue, des marquages représentatifs de volumes de manière à faciliter la préparation ou le remplissage du récipient 10 par un liquide.

L'embout 20 est prévu pour dispenser le liquide dans la narine d'un utilisateur. L'embout 20 comporte une embase 21 et un sommet, ou une buse, 22. L'orifice 23 destiné à permettre l'écoulement du liquide hors du dispositif 1 est pratiqué dans le sommet 22. L'orifice 23 est de section circulaire. L'embout 20 comporte encore un second orifice 24 raccordé à l'orifice d'écoulement 23 par un conduit interne 25 qui s'étend depuis l'embase 21 jusqu'à l'orifice 23. La section du conduit interne 25 décroit depuis l'embase 21 jusqu'à l'orifice 23. L'orifice 24 est ainsi aménagé à une extrémité du conduit 25 et l'orifice 23 à l'autre extrémité du conduit 25. De manière avantageuse, la section du conduit 25 est telle qu'elle permet le passage d'un goupillon de nettoyage et l'observation à l'oeil nu de l'aspect intérieur du conduit 25.

Le sommet 22 de l'embout 20 présente une surface convexe 26. La surface convexe 26 est prévue pour être apposée contre la narine d'un utilisateur adulte ou enfant. Dans un premier mode de réalisation, la surface convexe 26 est arrondie. Dans un second mode de réalisation, le sommet 22 présente une surface convexe 26 et non arrondie, c'est-à-dire que les parois de la surface convexe 26 sont prolongées par des parois avec lesquelles elles forment un angle (non représenté).

La surface convexe 26 et, préférentiellement, la totalité de l'embout 20, est réalisée dans un matériau qui permet d'éviter l'écoulement de liquide et qui procure une certaine adhérence au toucher à l'inverse d'un toucher d'un matériau totalement lisse. De plus le matériau est tel qu'il ne s'oppose pas à la rotation de l'embout 20 relativement à une pièce sur laquelle il est fixé comme expliqué dans ce qui suit. Il s'agit d'un matériau en élastomère, par exemple tel que celui commercialisé sous la marque commerciale Dryflex.

L'embout 20 comporte entre son embase 21 et son sommet 22 un ou plusieurs méplats 27 destinés à faciliter la préhension, chaque méplat 27 permettant d'apposer un doigt. Préférentiellement, l'embout présente deux méplats 27 permettant la prise de l'embout entre l'index et le pouce.

L'embase 21 comprend une partie cylindrique 212. Une partie tronconique 211 est prévue entre le sommet 22 et l'embase 21. La partie cylindrique 212 présente un diamètre inférieur à celui de la grande base de la partie tronconique 211.

Le dispositif 1 comprend encore une pièce intermédiaire 30 disposée entre le récipient 10 et l'embout 20. Le troisième orifice 36 traverse la pièce intermédiaire 30.

La pièce intermédiaire 30 se présente sous la forme d'une bague tronconique. Elle comprend une extrémité supérieure destinée à être reliée à l'embout 20 et une extrémité inférieure destinée à être reliée au récipient 10.

La pièce intermédiaire 30 comporte à cet effet des moyens pour réceptionner l'embout 20 et pour permettre la rotation de l'embout 20 relativement à la pièce intermédiaire 30. Les moyens prévus pour réceptionner l'embout 20 se composent d'un logement cylindrique 33 dans lequel peut être logée la partie cylindrique 212 constitutive de l'embase 21 de l'embout. Dans le mode de réalisation décrit à la Fig. 1, l'embout 20 est relié à la pièce intermédiaire 30 par clipage de l'embase 21 dans le logement cylindrique 33.

Des moyens d'étanchéité 40 sont prévus entre la paroi cylindrique du logement 33 et la paroi cylindrique de l'embase 21. Ces moyens d'étanchéité 40 comprennent deux bourrelets 41 et 42 formés autour de la paroi cylindrique 212 de l'embase 21. Les bourrelets 41 et 42 se trouvent contre la paroi cylindrique du logement 33 lorsque l'embout est connecté sur la pièce intermédiaire 30.

De manière à garantir la fixation de l'embout 20 sur la pièce intermédiaire 30, un moyen de retenue 50 est prévu entre l'embout 20 et la pièce intermédiaire 30. Ce moyen 50 peut être composé d'un bourrelet 501 formé autour de la paroi cylindrique du logement 33 ou encore de butées formées sur cette paroi.

Les moyens d'étanchéité 40 et les moyens de retenue 50 autorisent la rotation de l'embout 20 relativement à la pièce intermédiaire 30. Etant donné que l'embout 20 est fixé par clipage, ou par emboîtement, sans vissage, l'embout 20 entraîné en rotation dans le logement 33 ne coulisse pas par rapport à celui-ci.

La pièce intermédiaire 30 est solidarisée de manière provisoire et étanche sur le récipient 10. Celui-ci comprend un goulot 12 pourvu d'un filetage 121 complémentaire d'un taraudage 351 réalisé dans la pièce intermédiaire 30. De manière à garantir l'étanchéité de la fixation, la pièce intermédiaire 30 comporte une portion cylindrique 35 qui présente le taraudage 351 ainsi qu'une portion cylindrique 35b à l'intérieur de la portion 35 de manière à ce que le filetage 121 se retrouve pris en sandwich entre les portions cylindriques 35 et 35b lorsque la pièce intermédiaire 30 est fixée sur le récipient 10.

Le troisième orifice 36 est pratiqué dans une paroi de fond 37 de la pièce intermédiaire 30. La paroi de fond 37 est confondue avec le fond du logement cylindrique 33. L'orifice 36 est, de plus, pratiqué dans l'axe longitudinal de la pièce intermédiaire 30 si bien qu'il est directement en communication avec l'orifice 24 de l'embout 20. Le second orifice 24 et le troisième orifice 36 sont alignés et donc centrés dans un même axe Ax lorsque l'embout 20 est monté sur la pièce intermédiaire 30.

L'orifice 36 communique également avec l'ouverture 13 du récipient 10 par l'intermédiaire d'un conduit 38.

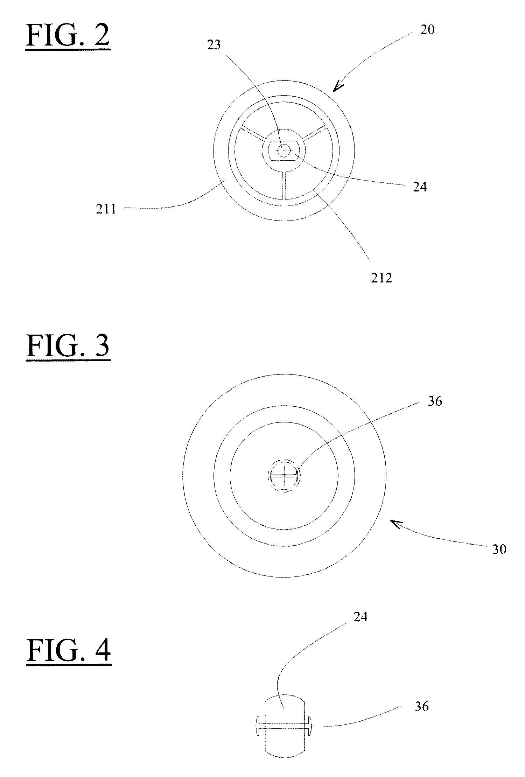

Les orifices 24 et 36 sont de section allongée (Figs. 2 et 3). Lorsque l'embout 20 est entraîné en rotation, par exemple manuellement par l'utilisateur, la section de passage du liquide varie, cette section traversant les second et troisième orifices 24 et 36. Il en résulte une modification du débit du flux de liquide qui traverse les orifices 24 et 36 entre une position selon laquelle les lumières des orifices 24 et 36 sont confondues entièrement et une position selon laquelle les lumières des orifices 24 et 36 ne se confondent pas complètement.

La section de l'orifice 24 est par exemple oblongue ou rectangulaire. La section de l'orifice 36 se présente par exemple sous la forme d'une section rectangulaire plus étroite que celle de l'orifice 24 et dont les extrémités présentent un renflement, la section de l'orifice 36 rappelant la forme d'un I (Figs. 3 et 4). La rotation de l'embout 20 permet alors de positionner les orifices 24 et 36 l'un relativement à l'autre soit de manière à ce que la totalité de la lumière de l'orifice 36 communique ou se confonde avec la lumière 24 (première position), soit de manière à ce que la lumière des renflements de la section rectangulaire de l'orifice 36 ne communique pas avec la lumière de l'orifice 24 (seconde position) (Fig. 4). La première position autorise un débit de liquide traversant les orifices 24 et 36 intense alors que la seconde position autorise un débit de liquide traversant les orifices 24 et 36 plus faible par rapport au premier débit.

Les orifices 24 et 36 étant centrés sur le même axe Ax comme cela est visible sur la Fig. 1, il s'en suit que le passage traversant ces deux orifices 24 et 36 n'est jamais fermé, quelque soit la rotation de l'embout 20 par rapport à la pièce intermédiaire 30

Il est à noter que les méplats 27 facilitent la prise en main de l'embout 20 et sa rotation relativement à la pièce intermédiaire 30 lorsque les doigts de l'utilisateur sont mouillés.

La section plus étroite de l'orifice 36 permet de conserver une section de l'orifice 24 suffisamment large pour permettre la visibilité à l'oeil nu de l'aspect du conduit 25 et son nettoyage, notamment pour permettre l'introduction d'un écouvillon dans le conduit 25.

D'autres sections sont envisageables concernant les orifices 24 et 36 dès lors qu'elles ne sont pas circulaires.

De manière à guider l'utilisateur dans le choix du débit, des indications visuelles relatives au réglage du débit du flux de liquide sont prévues. Par exemple, un marquage visuel 39 du débit et plus particulièrement de la position relative des orifices est prévu sur la pièce intermédiaire 30. Un marquage complémentaire 28 est prévu sur l'embout 20. La rotation de l'embout 20 relativement à la pièce intermédiaire 30 permet d'aligner en vis à vis le marquage 39 et le marquage 28 et de régler le flux selon un débit élevé ou faible, selon le cas.

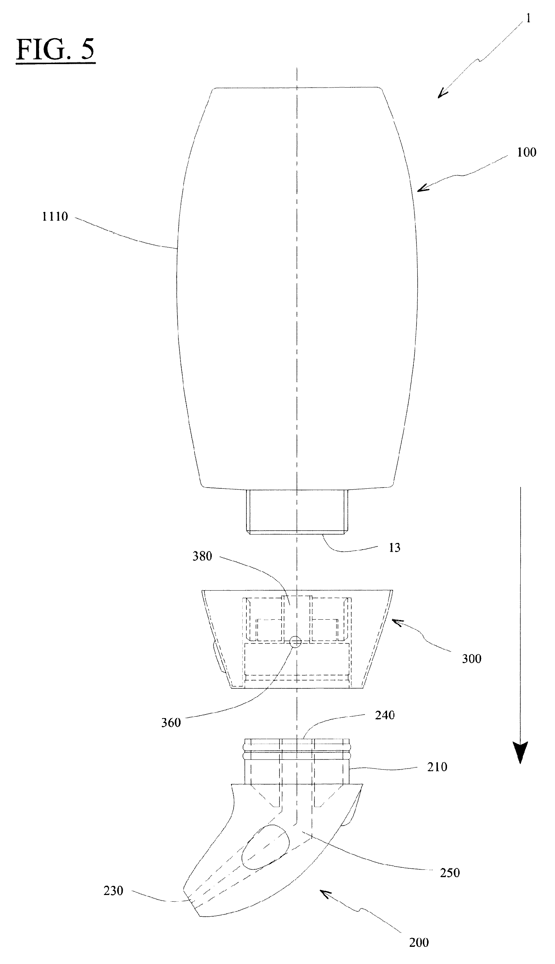

Deux types d'embouts 20 et 200 peuvent être connectés sur le dispositif 1 et plus particulièrement sur la pièce intermédiaire 30 (Figs. 1 et 5). Le choix de l'embout dépend du procédé d'irrigation choisi par l'utilisateur. En effet, le dispositif 1 offre le choix entre deux procédés, un premier procédé d'irrigation par ascension du liquide au travers d'un tube plongeur du dispositif 1, et un second procédé d'irrigation par écoulement par gravité du liquide au travers du dispositif 1.

Le premier procédé d'irrigation par ascension du liquide a lieu dans un sens opposé à la gravité et nécessite de faire communiquer un tube plongeur 60 avec l'orifice 36, le tube plongeur 60 plongeant à l'intérieur du récipient 10 et du liquide lorsque le récipient est rempli (voir flèche Fig. 1). Lors de ce procédé, le dispositif est orienté de manière à ce que l'embout 20 soit dirigé vers le haut. Le tube 60 est raccordé à l'orifice 36 par l'intermédiaire du conduit 38 sur lequel il est connecté, par exemple par force.

Le conduit 25 qui réunit l'orifice d'écoulement 23 et le second orifice 24 est droit en ce qui concerne l'embout 20 prévu pour la mise en oeuvre de ce premier procédé (Fig. 1). Le conduit 25 est conique. Les orifices 23, 24 et 36 sont centrés sur le même axe Ax.

Lors de la mise en oeuvre de ce premier procédé d'irrigation, le flux de liquide est généré par pression des parois 111, par exemple, par compression par la main. Le flux pénètre dans le tube plongeur 60 puis dans le conduit 38. Il traverse alors la section de passage générée par la position relative des orifices 24 et 36 et s'écoule jusqu'à l'orifice d'écoulement 23 par l'intermédiaire du conduit 25, puis s'écoule hors du dispositif 1 jusque dans la narine de l'utilisateur contre laquelle la partie convexe 26 doit être apposée. Le débit du flux peut être modifié de manière contrôlée après rotation de l'embout 20 relativement à la pièce intermédiaire 30, avec pour conséquence la rotation autour de son axe central de l'ouverture 24 et donc la variation des positions relatives des second et troisième orifices 24 et 36.

Ce premier procédé est préférentiellement adapté au cas de congestions fortes ou de symptômes naso-sinusiens sévères en cas de rhinites, de sinusites, de rhino sinusites chroniques ou de soins post-opératoires.

Le second procédé d'irrigation par gravité du liquide ne nécessite pas de tube plongeant à l'intérieur du récipient ou du liquide lorsque le récipient est rempli (Fig. 5). Le conduit 250 qui réunit l'orifice d'écoulement 230 et le second orifice 240 est coudé dans l'embout 200 prévu pour la mise en oeuvre de ce second procédé. L'embout 200 est en tout autre point identique à l'embout 20 précédemment décrit. Lors de ce procédé, le dispositif 1 est orienté avec l'embout 200 dirigé vers le bas.

Lors de la mise en oeuvre de ce second procédé d'irrigation, le flux de liquide est généré par pression des parois 1110 du récipient 110, par exemple, par compression par la main. Il en résulte qu'en absence de compression des parois 1110, aucune fuite massive de liquide ne s'observe lorsque le dispositif est orienté l'embout 200 en bas. La section plus étroite de l'orifice 36 en forme de I contribue également à ce fait. Le flux de liquide descend alors par gravité dans le conduit 380 de la pièce intermédiaire 300 (voir flèche Fig. 5). Il traverse ensuite la section de passage générée par la position relative des orifices 240 et 360 et s'écoule jusqu'à l'orifice d'écoulement 230 par l'intermédiaire du conduit 250, puis s'écoule hors du dispositif 1 jusque dans la narine de l'utilisateur contre laquelle la partie convexe 26 doit être apposée. Là encore, le débit du flux peut être modifié de manière contrôlée par la variation des positions relatives des second et troisième orifices 240 et 360 après rotation de l'embout 200.

Ce second procédé est préférentiellement adapté au cas de prévention des rhinites, sinusites ou rhino sinusites ou encore comme procédé de nettoyage quotidien hors périodes de pathologies ou en cas de symptômes naso-sinusiens modérés.

Après utilisation, le démontage du dispositif 1 est très rapide et aisé. Le récipient 10 ou 100, la pièce intermédiaire 30 ou 300 et l'embout 20 ou 200 sont séparés facilement. En particulier, les conduits 25 ou 250 des embouts 20 ou 200 sont nettoyés à l'aide d'un goupillon et avec de l'eau savonneuse, du vinaigre blanc ou de l'alcool à 70°. L'assemblage des pièces est également simplifié du fait qu'elles ne sont qu'au nombre de trois : le récipient, la pièce intermédiaire et l'embout. L'oublie d'une pièce dans le montage est exclu.

## Revendications

1. Dispositif d'irrigation des cavités naso-sinusiennes (1) comprenant un récipient (10), un embout (20, 200) comportant une embase (21) prévue pour être fixée sur ledit récipient (10) et un sommet (22) présentant un orifice (23) destiné à permettre l'écoulement du liquide hors du dispositif (1), et un moyen de réglage du débit du flux dudit liquide susceptible de s'écouler au travers dudit orifice d'écoulement (23), **caractérisé en ce que** :
- le récipient (10) comporte un corps (11) aux parois (111) flexibles, un fond plat et une ouverture (13) aménagée dans un goulot (12),
- l'embout (20) est traversé par un conduit (25, 250) présentant à chacune de ses extrémités un orifice (23, 24), dont un premier orifice (23) étant l'orifice destiné à permettre
l'écoulement du liquide hors du dispositif (1), et un second orifice (24) présentant une section allongée,
- une pièce intermédiaire (30) apte à être solidarisée avec ledit récipient (10) est disposée entre le récipient (10) et l'embout (30), fixée sur le goulot (12), et traversée par un conduit (38) débouchant sur un troisième orifice (36) présentant une section allongée, le second orifice (24) et le troisième orifice (36) étant alignés dans un même axe (Ax) lorsque l'embout (20) est monté sur ladite pièce intermédiaire (30),
- le moyen de réglage du débit du flux dudit liquide susceptible de s'écouler au travers dudit orifice d'écoulement (23) est composé de moyens prévus pour entrainer en rotation ledit embout (20) et faire varier les positions relatives desdits second et troisième orifices (24, 36) de sorte que la section de passage de liquide traversant les second et troisième orifices (24, 36) puisse varier autour de l'axe (Ax), la section du troisième orifice (36) est plus étroite que la section du second orifice (24).

2. Dispositif (1) selon la revendication 1, **caractérisé en ce que** ladite pièce intermédiaire (30) comporte des moyens pour réceptionner l'embout (33) et pour permettre la rotation dudit embout (20) relativement à ladite pièce intermédiaire (30).

3. Dispositif (1) selon l'une des revendications 1 à 2, **caractérisé en ce que** ladite pièce intermédiaire (30) comporte des moyens pour réceptionner et pour solidariser (121, 351) le récipient (10) de manière étanche.

4. Dispositif (1) l'une des revendications 2 à 3, **caractérisé en ce que** les moyens prévus pour réceptionner l'embout (20, 200) se composent d'un logement cylindrique (33) dans lequel peut être logée une partie cylindrique (212) constitutive de l'embase (21) dudit embout (20).

5. Dispositif (1) selon la revendication 4, **caractérisé en ce que** des moyens d'étanchéité (40) sont prévus entre une paroi cylindrique du logement cylindrique (33) et une paroi cylindrique de l'embase (21).

6. Dispositif (1) selon la revendication 5, **caractérisé en ce que** les moyens d'étanchéité (40) comprennent au moins un bourrelet (41, 42) formé autour de la paroi cylindrique de l'embase (21) apte à coopérer avec la paroi cylindrique constitutive dudit logement (33).

7. Dispositif (1) selon l'une des revendications 1 à 6, **caractérisé en ce qu'**au moins un moyen de retenue (501) est prévu entre l'embout (20) et la pièce intermédiaire (30).

8. Dispositif (1) selon l'une des revendications 1 à 7, **caractérisé en ce que** ledit conduit (25) qui réunit l'orifice d'écoulement (23) et le second orifice (24) est droit.

9. Dispositif (1) selon l'une des revendications 1 à 7, **caractérisé en ce que** ledit conduit (25) qui réunit l'orifice d'écoulement (230) et le second orifice (240) est coudé.

10. Dispositif (1) selon l'une des revendications 1 à 9, **caractérisé en ce que** le récipient (10) comprend un goulot (12) pourvu d'un filetage (121) complémentaire d'un taraudage (351) réalisé dans la pièce intermédiaire (30).

11. Dispositif (1) selon l'une des revendications précédentes, **caractérisé en ce qu'**il comprend un tube plongeur (60) prévu pour être raccordé audit troisième orifice (36) et pour plonger dans ledit récipient (10).

12. Dispositif (1) selon l'une des revendications 2 à 11, **caractérisé en ce que** le troisième orifice (36) est pratiqué dans la paroi de fond (37) de ladite pièce intermédiaire (30).

13. Dispositif (1) selon l'une des revendications précédentes, **caractérisé en ce que** le sommet (22) dudit embout (20) présente une surface convexe.

14. Dispositif (1) selon l'une des revendications précédentes, **caractérisé en ce que** ledit embout (20) comporte au moins un méplat (27) entre l'embase (21) et le sommet (22).

15. Dispositif (1) selon l'une des revendications 1 à 14, **caractérisé en ce que** ladite pièce intermédiaire (30) comporte des indications visuelles (39) relatives au réglage du débit du flux de liquide.

16. Dispositif (1) selon l'une des revendications précédentes, **caractérisé en ce que** ledit embout (20) comporte une ou plusieurs indications visuelles (28) relatives au réglage du débit du flux de liquide.

17. Dispositif selon l'une des revendications précédentes, **caractérisé en ce que** le sommet (22) dudit embout (20) ou la partie convexe (26) présente une surface adhérente et non collante.

18. Kit comportant un dispositif (1) tel que défini dans l'une des revendications précédentes et comportant un embout (20) dont le conduit (25) qui réunit l'orifice d'écoulement (23) et le second orifice (24) est droit ainsi qu'un embout (200) dont le conduit (250) qui réunit l'orifice d'écoulement (230) et le second orifice (240) est coudé.

## Patentansprüche

1. Vorrichtung zur Spülung der Nasennebenhöhlen (1), umfassend einen Behälter (10), einen Ansatz (20, 200), umfassend einen Sockel (21), der dazu vorgesehen ist, auf dem Behälter (10) befestigt zu werden, und eine Spitze (22), die eine Öffnung (23) aufweist, die dazu bestimmt ist, das Fließen der Flüssigkeit aus der Vorrichtung (1) zu ermöglichen, und ein Mittel zur Einstellung der Durchflussmenge der Flüssigkeit, die durch die Abflussöffnung (23) fließen kann, **dadurch gekennzeichnet, dass**:
- der Behälter (10) einen Körper (11) mit biegsamen Wänden (111), einen flachen Boden und eine Öffnung (13) umfasst, die in einem Hals (12) angeordnet ist,
- der Ansatz (20) von einer Leitung (25, 250) durchquert wird, die an jedem ihrer Enden eine Öffnung (23, 24) aufweist, wobei eine erste Öffnung (23) die Öffnung ist, die dazu bestimmt ist, das Fließen der Flüssigkeit aus der Vorrichtung (1) zu ermöglichen, und eine zweite Öffnung (24) einen länglichen Querschnitt aufweist,
- ein Zwischenstück (30), das geeignet ist, mit dem Behälter (10) verbunden zu werden, zwischen dem Behälter (10) und dem Ansatz (30) angeordnet, auf dem Hals (12) befestigt ist und von einer Leitung (38) durchquert wird, die an einer dritten Öffnung (36) mündet, die einen länglichen Querschnitt aufweist, wobei die zweite Öffnung (24) und die dritte Öffnung (36) in einer selben Achse (Ax) ausgerichtet sind, wenn der Ansatz (20) auf dem Zwischenstück (30) montiert ist,
- das Mittel zur Regelung der Durchflussmenge der Flüssigkeit, die geeignet ist, durch die Abflussöffnung (23) zu fließen, aus Mitteln besteht, die dazu vorgesehen sind, den Ansatz (20) in Drehung anzutreiben und die relativen Positionen der zweiten und dritten Öffnung (24, 36) zu variieren, so dass der Flüssigkeitsdurchgangsquerschnitt durch die zweite und dritte Öffnung (24, 36) um die Achse (Ax) variieren kann, wobei der Querschnitt der dritten Öffnung (36) kleiner als der Querschnitt der zweiten Öffnung (24) ist.

2. Vorrichtung (1) nach Anspruch 1, **dadurch gekennzeichnet, dass** das Zwischenstück (30) Mittel umfasst, um den Ansatz (33) aufzunehmen und die Drehung des Ansatzes (20) in Bezug auf das Zwischenstück (30) zu ermöglichen.

3. Vorrichtung (1) nach einem der Ansprüche 1 bis 2, **dadurch gekennzeichnet, dass** das Zwischenstück (30) Mittel umfasst, um den Behälter (10) aufzunehmen und dicht zu verbinden (121, 351).

4. Vorrichtung (1) nach einem der Ansprüche 2 bis 3, **dadurch gekennzeichnet, dass** die Mittel, die dazu vorgesehen sind, den Ansatz (20, 200) aufzunehmen, aus einer zylindrischen Lagerung (33) bestehen, in der ein zylindrischer Teil (212), aus dem der Sockel (21) des Ansatzes (20) besteht, angeordnet sein kann.

5. Vorrichtung (1) nach Anspruch 4, **dadurch gekennzeichnet, dass** Dichtungsmittel (40) zwischen einer zylindrischen Wand der zylindrischen Lagerung (33) und einer zylindrischen Wand des Sockels (21) vorgesehen sind.

6. Vorrichtung (1) nach Anspruch 5, **dadurch gekennzeichnet, dass** die Dichtungsmittel (40) mindestens einen Wulst (41, 42) umfassen, der um die zylindrische Wand des Sockels (21) ausgebildet und geeignet ist, mit der zylindrischen Wand, aus der die Lagerung (33) besteht, zusammenzuwirken.

7. Vorrichtung (1) nach einem der Ansprüche 1 bis 6, **dadurch gekennzeichnet, dass** mindestens ein Haltemittel (501) zwischen dem Ansatz (20) und dem Zwischenstück (30) vorgesehen ist.

8. Vorrichtung (1) nach einem der Ansprüche 1 bis 7, **dadurch gekennzeichnet, dass** die Leitung (25), die die Abflussöffnung (23) und die zweite Öffnung (24) verbindet, gerade ist.

9. Vorrichtung (1) nach einem der Ansprüche 1 bis 7, **dadurch gekennzeichnet, dass** die Leitung (25), die die Abflussöffnung (230) und die zweite Öffnung (240) verbindet, gebogen ist.

10. Vorrichtung (1) nach einem der Ansprüche 1 bis 9, **dadurch gekennzeichnet, dass** der Behälter (10) einen Hals (12) umfasst, der mit einem Gewinde (121) versehen ist, das zu einem Gewinde (351), das in dem Zwischenstück (30) hergestellt ist, komplementär ist.

11. Vorrichtung (1) nach einem der vorhergehenden Ansprüche, **dadurch gekennzeichnet, dass** sie ein Tauchrohr (60) umfasst, das dazu vorgesehen ist, an die dritte Öffnung (36) angeschlossen zu werden und in den Behälter (10) einzutauchen.

12. Vorrichtung (1) nach einem der Ansprüche 2 bis 11, **dadurch gekennzeichnet, dass** die dritte Öffnung (36) in die Bodenwand (37) des Zwischenstücks (30) eingearbeitet ist.

13. Vorrichtung (1) nach einem der vorhergehenden Ansprüche, **dadurch gekennzeichnet, dass** die Spitze (22) des Ansatzes (20) eine konvexe Fläche aufweist.

14. Vorrichtung (1) nach einem der vorhergehenden Ansprüche, **dadurch gekennzeichnet, dass** der Ansatz (20) mindestens eine Abflachung (27) zwischen dem Sockel (21) und der Spitze (22) umfasst.

15. Vorrichtung (1) nach einem der Ansprüche 1 bis 14, **dadurch gekennzeichnet, dass** das Zwischenstück (30) Sichtangaben (39) in Zusammenhang mit der Regelung der Flüssigkeitsdurchflussmenge umfasst.

16. Vorrichtung (1) nach einem der vorhergehenden Ansprüche, **dadurch gekennzeichnet, dass** der Ansatz (20) eine oder mehrere Sichtangaben (28) in Zusammenhang mit der Regelung der Flüssigkeitsdurchflussmenge umfasst.

17. Vorrichtung nach einem der vorhergehenden Ansprüche, **dadurch gekennzeichnet, dass** die Spitze (22) des Ansatzes (20) oder der konvexe Teil (26) eine haftende und nicht klebende Fläche aufweist.

18. Bausatz, umfassend eine Vorrichtung (1), wie in einem der vorhergehenden Ansprüche definiert, und umfassend einen Ansatz (20), dessen Leitung (25), die die Abflussöffnung (23) und die zweite Öffnung (24) verbindet, gerade ist, sowie einen Ansatz (200), dessen Leitung (250), die die Abflussöffnung (230) und die zweite Öffnung (240) verbindet, gebogen ist.

## Claims

1. Device for irrigating the naso-sinal cavities (1) comprising a container (10), an end-piece (20, 200) comprising a base (21) designed to be fixed to the said container (10) and a tip (22) having an orifice (23) intended to allow the liquid to flow out of the device (1), and a means for regulating the flow rate of the stream of the said liquid capable of flowing through the said flow orifice (23), **characterized in that**:
- the container (10) comprises a body (11) with flexible walls (111), a flat bottom and an opening (13) formed in a neck (12),
- the end-piece (20) has passing through it a duct (25, 250) having, at each of its ends, an orifice (23, 24) of which a first orifice (23) is the orifice intended to allow the liquid to flow out of the device (1) and a second orifice (24) has an elongate cross section,
- an intermediate component (30) able to be secured to the said container (10) is arranged between the container (10) and the end-piece (30), is fixed to the neck (12) and has, passing through it, a duct (38) opening into a third orifice (36) of elongate cross section, the second orifice (24) and the third orifice (36) being aligned along one and the same axis (Ax) when the end-piece (20) is mounted on the said intermediate component (30),
- the means of regulating the flow rate of the stream of the said liquid capable of flowing through the said flow orifice (23) is made up of means intended to turn the said end-piece (20) and vary the relative positions of the said second and third orifices (24, 36) so that the cross section for the passage of liquid passing through the second and third orifices (24, 36) can vary about the axis (Ax), the cross section of the third orifice (36) being narrower than the cross section of the second orifice (24).

2. Device (1) according to Claim 1, **characterized in that** the said intermediate component (30) comprises means for accommodating the end piece (33) and for allowing the said end-piece (20) to turn relative to the said intermediate component (30).

3. Device (1) according to one of Claims 1 and 2, **characterized in that** the said intermediate component (30) comprises means (121, 351) for receiving and securing the container (10) in a fluidtight manner.

4. Device (1) according to one of Claims 2 and 3, **characterized in that** the means provided to receive the end-piece (20, 200) are made up of a cylindrical housing (33) in which a cylindrical constituent part (212) of the base (21) of the said end-piece (20) can be housed.

5. Device (1) according to Claim 4, **characterized in that** sealing means (40) are provided between a cylindrical wall of the cylindrical housing (33) and a cylindrical wall of the base (21).

6. Device (1) according to Claim 5, **characterized in that** sealing means (40) comprise at least one bulge (41, 42) formed around the cylindrical wall of the base (21) and able to collaborate with the cylindrical wall that makes up the said housing (33).

7. Device (1) according to one of Claims 1 to 6, **characterized in that** at least one retaining means (501) is provided between the end-piece (20) and the intermediate component (30).

8. Device (1) according to one of Claims 1 to 7, **characterized in that** the said duct (25) which connects the flow orifice (23) and the second orifice (24) is straight.

9. Device (1) according to one of Claims 1 to 7, **characterized in that** the said duct (25) connecting the flow orifice (230) and the second orifice (240) is bent.

10. Device (1) according to one of Claims 1 to 9, **characterized in that** the container (10) comprises a neck (12) provided with a screw thread (121) that complements a tapped thread (351) made in the intermediate component (30).

11. Device (1) according to one of the preceding claims, **characterized in that** it comprises a dip tube (60) intended to be connected to the said third orifice (36) and to dip down inside the container (10).

12. Device (1) according to one of Claims 2 to 11, **characterized in that** the third orifice (36) is made in the bottom wall (37) of the said intermediate component (30).

13. Device (1) according to one of the preceding claims, **characterized in that** the top (22) of the said end-piece (20) has a convex surface.

14. Device (1) according to one of the preceding claims, **characterized in that** the said end-piece (20) comprises at least one flat (27) between the base (21) and the top (22).

15. Device (1) according to one of Claims 1 to 14, **characterized in that** the said intermediate component (30) comprises visual indications (39) relating to the regulation of the flow rate of the stream of liquid.

16. Device (1) according to one of the preceding claims, **characterized in that** the said end-piece (20) comprises one or several visual indications (28) relating to the regulation of the flow rate of the stream of liquid.

17. Device according to one of the preceding claims, **characterized in that** the top (22) of said end-piece (20), or the convex part (26) has a grippy and non-sticky surface.

18. Kit comprising a device (1) as defined in one of the preceding claims and comprising an end-piece (20) of which the duct (25) which connects the flow orifice (23) and the second orifice (24) is straight and an end-piece (200) of which the duct (250) which connects the flow orifice (230) and the second orifice (240) is bent.
